# EUROPEAN PATENT APPLICATION

(11) **EP 4 721 655 A1**
(43) Date of publication of application: **08.04.2026**
(21) Application number: 24203921.2
(22) Date of filing: 01.10.2024
(51) Int. Cl.: A61B 5/024, A61B 5/1455, A61B 5/00

(54) **PHOTOPLETHYSMOGRAPHY DEVICE OPTICAL COUPLING**

(71) Applicant: Koninklijke Philips N.V., 5656 AG Eindhoven (NL)
(72) Inventor: PAULUSSEN, Elvira Johanna Maria, Eindhoven (NL); SCHWENK, Marcus, Eindhoven (NL)
(74) Representative: Philips Intellectual Property & Standards

(57) **Abstract**

An adhesive patch (100) for providing optical coupling between body tissue (110) and a photoplethysmography device (120), is provided. The adhesive patch includes: a first surface (130) for coupling to the body tissue (110), the first surface comprising an adhesive material for providing temporary coupling to the body tissue (110). The adhesive patch also includes a second surface (140) for coupling to the photoplethysmography device (120), the second surface being arranged opposite the first surface. The adhesive patch also includes a transparent region (150). The transparent region (150) extends between the first surface (130) and the second surface (140) for providing the optical coupling between the body tissue (110) and the photoplethysmography device (120) when the first surface is coupled to the body tissue and the second surface is coupled to the photoplethysmography device.

## Description

### FIEL OF THE INVENTION

The present disclosure relates to providing optical coupling between body tissue and a photoplethysmography device. An adhesive patch, a photoplethysmography device, and a patient monitoring system, are disclosed.

### BACKGROUND OF THE INVENTION

Photoplethysmography, or "PPG", is an optical technique for measuring blood volume changes in body tissue. Photoplethysmography is used to provide physiological information relating to the health of a subject. For instance, PPG may be used to provide measurements of a subject's blood oxygen saturation, heart rate, respiration rate, and also to determine information relating to their sympathetic nervous system activity and thermoregulation.

PPG may be performed using various portions of the optical spectrum. Some types of physiological information are derivable from PPG measurements that are acquired over a single wavelength interval. For instance, a measurement of a subject's heart rate is typically derived from PPG measurements within a wavelength interval that corresponds to a red or a green portion of the optical spectrum. Other types of physiological information are typically derived from PPG measurements that are acquired over multiple wavelength intervals. For instance, measurements of blood oxygen saturation are typically derived from PPG measurements that are acquired over wavelength intervals that correspond to both the red and the infrared portions of the optical spectrum. Blood oxygen saturation measurements are typically derived from the ratio of pulse amplitudes of red and infrared PPG measurements (e.g. approximately 660 nm and approximately 880 nm or 940 nm, respectively) using the "ratio of ratios" technique. This metric quantifies the difference in optical absorption in tissue between oxygen-bound and oxygen-unbound haemoglobin at red versus infrared wavelengths. A measurement of blood oxygen saturation may be referred-to as an "SpO₂ level.

PPG measurements may be acquired from body tissue at various locations in the anatomy. For instance, PPG measurements may be acquired from body tissue in a finger, or a wrist, forehead, or an ear lobe, or a nostril, of a subject. PPG measurements are acquired using a PPG device. A PPG device includes one or more optical sources and one or more optical detectors. The optical source(s) irradiate the body tissue with optical radiation, and the optical detector(s) detect an amount of the optical radiation that is returned from the tissue in response to the irradiation. PPG measurements can be acquired in both a transmissive mode wherein the PPG device detects an amount of the optical irradiation that is transmitted by the body tissue, or in a reflective mode wherein the PPG device detects an amount of the optical irradiation that is reflected and/or scattered by the body tissue. A combination of these measurements may also be used.

The acquisition of reliable PPG measurements depends in-part on maximising the amount of optical radiation that is coupled between the optical source(s) and the body tissue, and also maximising the amount of optical radiation that is coupled between the body tissue and the optical detector(s). Any leakage of optical radiation at these interfaces risks degrading the performance of the PPG device. For instance, it may degrade the signal-to-noise "SNR" ratio of a PPG measurement. In-turn, this degrades the accuracy of the PPG measurement or it requires the use of increased power by the optical source(s) in order to compensate for the leakage. Any leakage of optical radiation at the interface between the optical source(s) and the body tissue also risks being detected by the optical detector(s) without having passed through tissue. This is undesirable because it consumes the dynamic range of the optical detector, which in-turn degrades its sensitivity and/or places additional requirements on the processing of PPG measurements. However, it is challenging to provide good optical coupling with the body at these interfaces. This is due to the uneven nature of the skin, and also disruptions at these interfaces arising from the subject's movement. Such disruptions can result in inaccurate PPG measurements or an absence of PPG measurements. Existing PPG devices typically address these issues by applying pressure between the PPG device and the body tissue at these interfaces. This has the drawback of reducing the amount of blood flow in the vicinity of these interfaces, which reduces the accuracy of the PPG measurements. Thus, there remains a need to further improve the optical coupling between body tissue and PPG devices in order to further improve their performance.

### SUMMARY OF THE INVENTION

The invention is defined by the claims.

According to one aspect of the present disclosure, an adhesive patch for providing optical coupling between body tissue and a photoplethysmography device, is provided. The adhesive patch includes:
a first surface for coupling to the body tissue, the first surface comprising an adhesive material for providing temporary coupling to the body tissue;
a second surface for coupling to the photoplethysmography device, the second surface being arranged opposite the first surface; and
a transparent region;
wherein the transparent region extends between the first surface and the second surface for providing the optical coupling between the body tissue and the photoplethysmography device when the first surface is coupled to the body tissue and the second surface is coupled to the photoplethysmography device.

The adhesive patch therefore provides optical coupling between the body tissue and the photoplethysmography device. The first surface of the adhesive patch includes an adhesive material for providing temporary coupling to the body tissue. The patch can therefore be temporarily coupled to the body. This improves the stability of the optical coupling between the body tissue and the photoplethysmography device because it reduces relative movement between the body tissue and the photoplethysmography device when a subject moves. It therefore reduces disruptions at this interface that might otherwise arise from a subject's movements and result in inaccurate PPG measurements or an absence of PPG measurements.

In accordance with one aspect of the present disclosure, the transparent region of the adhesive patch comprises a refractive index value in the range from approximately 1.2 to 1.6. Body tissue typically has a refractive index of approximately 1.4. By providing the transparent region with a refractive index within this range, Fresnel reflection at the interface between the transparent region and body tissue is reduced. Fresnel reflection that might otherwise occur at this interface would reduce the amount of optical radiation emitted by an optical source of the photoplethysmography device that reaches an optical detector of the photoplethysmography device. Moreover, optical sources and optical detectors are typically formed from materials that have a refractive index of approximately 1.5, and so this range also serves to reduce Fresnel reflection at the interface between the transparent region and the optical source or detector. Consequently, providing the transparent region of the adhesive patch with a refractive index value in this range serves to improve the SNR of PPG measurements that are acquired using the photoplethysmography device.

In accordance with one aspect of the present disclosure, the first surface and/or the second surface of the adhesive patch comprises a raised profile within at least a portion of the transparent region. The raised profile defines a lens for coupling optical radiation between the body tissue and the photoplethysmography device, or vice versa, when the first surface is coupled to the body tissue and the second surface is coupled to the photoplethysmography device. In this aspect, the lens serves to improve the coupling of the optical radiation between the body tissue and the photoplethysmography device. The lens may concentrate optical radiation emitted by an optical source of the photoplethysmography device onto the body tissue and/or concentrate optical radiation emitted by the body tissue onto an optical detector of the photoplethysmography device, for example.

In accordance with one aspect of the present disclosure, the second surface of the adhesive patch comprises a raised profile within the at least a portion of the transparent region. The raised profile is configured to mate with a corresponding recess in a surface of the photoplethysmography device for aligning the adhesive patch with the photoplethysmography device. The mating that is provided by the raised profile and the corresponding recess serves to improve the stability of the optical coupling between the body tissue and the photoplethysmography device. Improving the stability of the optical coupling reduces disruptions at this interface that might otherwise arise from a subject's movements and result in inaccurate PPG measurements or an absence of PPG measurements.

In accordance with one aspect of the present disclosure, the raised profile further defines a lens for coupling optical radiation between the body tissue and the photoplethysmography device, or vice versa, when the first surface is coupled to the body tissue and the second surface is coupled to the photoplethysmography device. In this aspect, the raised profile serves to improve the stability of the optical coupling, as well as to provide a lens that further improves the coupling of the optical radiation between the body tissue and the photoplethysmography device.

In accordance with one aspect of the present disclosure, the second surface of the adhesive patch comprises one or more alignment features. The one or more alignment features are configured to mate with one or more corresponding alignment features on the photoplethysmography device for aligning the adhesive patch with the photoplethysmography device. The mating that is provided by the alignment features and the alignment features serves to improve the stability of the optical coupling between the body tissue and the photoplethysmography device. Improving the stability of the optical coupling reduces disruptions at this interface that might otherwise arise from a subject's movements and result in inaccurate PPG measurements or an absence of PPG measurements.

In accordance with one aspect of the present disclosure, the adhesive patch includes a first border. The first border at least partially surrounds the transparent region. The first border comprises a reflective and/or scattering material for returning optical radiation incident on the first border to the body tissue when the first surface is coupled to the body tissue. The first border recycles optical radiation that escapes from the skin by instead returning it to the skin and providing the optical radiation with another opportunity to reach an optical detector of the photoplethysmography device. The recycled optical radiation may therefore contribute to a PPG measurement, which improves the SNR of the PPG measurement.

In accordance with one aspect of the present disclosure, the first border defines at least a portion of the first surface and/or the second surface. In some configurations, the first border may define a portion of the first surface and/or the second surface, and in other configurations the first border may be set back from the first and/or second surface. In the former case, the amount of recycled optical radiation may be improved. In the latter case, manufacturability of the adhesive patch may be improved.

In accordance with one aspect of the present disclosure, the first border of the adhesive patch comprises one or more inner sidewalls contacting the transparent region. The one or more inner sidewalls define an aperture for optical radiation passing through the transparent region. The aperture is a tapered aperture having a relatively larger size at the first surface than at the second surface. Alternatively or additionally, the one or more inner sidewalls comprise a reflective material. The tapered aperture, and likewise the reflective material serves to guide optical radiation between the body tissue and the photoplethysmography device. The tapered aperture may additionally serve to concentrate optical radiation onto an optical detector of the photoplethysmography device, or to provide a desired distribution in the body tissue of the optical radiation emitted by an optical source of the photoplethysmography device.

In accordance with one aspect of the present disclosure, the adhesive patch includes a second border. The second border at least partially surrounds the transparent region and/or the first border. The second border comprises an optically absorbing material. The second border serves to absorb optical radiation emitted by the optical source of the photoplethysmography device that either escapes from the body tissue, or does not pass through the body tissue at all. Some of the optical radiation that escapes from the body tissue includes pulsatile information relating to blood volume changes in the body tissue. This optical radiation has typically passed through body tissue in which there is a significant amount of blood flow. This may be described as functional optical radiation because it includes physiological information relating to the body. By contrast, some of the optical radiation that escapes from the body tissue does not include pulsatile information. This latter optical radiation has typically passed through portions of the body tissue such as bone, nail, and so forth, and in which there is an insignificant amount of blood flow. This may be described as non-functional optical radiation. Some optical radiation does not pass through the body tissue at all and is instead guided between the optical source and an optical detector of the photoplethysmography device, e.g. via reflection and/or scattering between a housing of the photoplethysmography device and a surface of the body tissue. This may be described as non-functional optical radiation because it does not include physiological information relating to the body. The detection of non-functional optical radiation is undesirable because it consumes the dynamic range of the optical detector, which in-turn degrades its sensitivity and/or places additional requirements on the processing of PPG measurements. The optically absorbing material in this aspect serves to absorb both the non-functional optical radiation that escapes from the body tissue, and the non-functional optical radiation that does not pass through the body tissue at all. This preserves the dynamic range of the optical detector, and in-turn avoids the degraded sensitivity and/or the additional processing requirements of the PPG measurements.

In accordance with one aspect of the present disclosure, the second border of the adhesive patch defines at least a portion of the first surface and/or the second surface. In some configurations, the second border may define a portion of the first surface and/or the second surface, and in other configurations the second border may be set back from the first and/or second surface. In the former case, the amount of absorbed optical radiation may be improved. In the latter case, manufacturability of the adhesive patch may be improved.

In accordance with one aspect of the present disclosure, the transparent region of the adhesive patch is configured to provide optical coupling between a first region of the body tissue and an optical source of the photoplethysmography device when the first surface is coupled to the body tissue and the second surface is coupled to the photoplethysmography device. The adhesive patch also includes a second transparent region and an optically absorbing region. The second transparent region extends between the first surface and the second surface for providing optical coupling between a second region of the body tissue and an optical detector of the photoplethysmography device when the first surface is coupled to the body tissue and the second surface is coupled to the photoplethysmography device. The optically absorbing region is arranged between the transparent region and the second transparent region for separating the optical coupling provided by the transparent region from the optical coupling provided by the second transparent region. By separating the optical coupling that is provided by the transparent region from the optical coupling that is provided by the second transparent region, the optically absorbing region reduces the amount of non-functional optical radiation that is detected by an optical detector of the photoplethysmography device.

In accordance with one aspect of the present disclosure, the second transparent region comprises a refractive index value in the range from 1.2 to 1.6. As mentioned above, body tissue typically has a refractive index of approximately 1.4. Providing the transparent region with a refractive index within this range, similarly serves to reduce Fresnel reflection at the interface between the transparent region and tissue. This in-turn serves to improve the SNR of PPG measurements that are acquired using the photoplethysmography device.

In accordance with one aspect of the present disclosure, the adhesive patch is configured to be wrapped around at least a portion of a finger. The adhesive patch may have a size, a conformability, and a shape that facilitates its wrapping around a finger, for example. The adhesive patch may have a flat shape in the resting state, or a curved shape in the resting state, for example. The adhesive patch may therefore be used to obtain PPG measurements from a subject's finger.

In accordance with one aspect of the present disclosure, the adhesive patch is configured to be wrapped around the at least a portion of a finger such that the transparent region is arranged on one side of the finger and such that the second transparent region is arranged on an opposing side of the finger for making an optical transmission measurement through the finger with the photoplethysmography device. The adhesive patch may therefore be used to obtain PPG measurements from a subject's finger in a transmissive mode.

In accordance with one aspect of the present disclosure, at least one of the following is formed from a polymer material: the transparent region, the second transparent region, the first border, the second border. Polymer materials such as polydimethylsiloxane "PDMS" or liquid silicone rubbers "LSR" may be used, for example. Other examples of polymers that may be used include Hydrogels, Chitin, and Chitosan.

In accordance with one aspect of the present disclosure, at least one of the following is formed from a flexible material: the transparent region, second transparent region, the first border, the second border. Forming these elements from a flexible material allows them to conform to a surface of the body tissue. This improves the optical coupling between the body tissue and the photoplethysmography device because it limits the extent of an air gap that might otherwise form between the surface of the body tissue and the photoplethysmography device. Fresnel reflections may occur at this air gap and consequently reduce the amount of optical radiation emitted by an optical source of the photoplethysmography device that reaches an optical detector of the photoplethysmography device. Consequently, forming these elements from a flexible material serves to improve the SNR of PPG measurements that are acquired using the photoplethysmography device.

In accordance with one aspect of the present disclosure, the second surface of the adhesive patch comprises an adhesive material for providing temporary coupling to the photoplethysmography device. The adhesive material improves the stability of the optical coupling between the body tissue and the photoplethysmography device because it reduces relative movement between the body tissue and the photoplethysmography device when a subject moves. This reduces disruptions at this interface that might otherwise arise from a subject's movements and result in inaccurate PPG measurements or an absence of PPG measurements.

In accordance with one aspect of the present disclosure, the adhesive material of the first surface covers at least a portion of the transparent region and/or the adhesive material of the second surface covers at least a portion of the transparent region. Covering a portion these regions with adhesive material serves to limit the extent of an air gap that might otherwise form between the surface of the body tissue and these regions of the adhesive patch. Fresnel reflections that might otherwise occur at this air gap would reduce the amount of optical radiation emitted by an optical source of the photoplethysmography device that reaches an optical detector of the photoplethysmography device. Thus, covering a portion these regions with adhesive material serves to improve the SNR of PPG measurements that are acquired using the photoplethysmography device.

In accordance with one aspect of the present disclosure, the transparent region of the adhesive patch comprises a refractive index in the range from 1.2 to 1.6 at one or more wavelengths in the range from approximately 520nm to 1000nm. As mentioned above, body tissue typically has a refractive index of approximately 1.4. By providing the transparent region with a refractive index within this range, Fresnel reflection at the interface between the transparent region and body tissue is reduced. This in-turn improves the SNR of PPG measurements that are acquired using the photoplethysmography device. Moreover, wavelengths in the range from approximately 520nm to 1000nm are often used for PPG measurements, and so the use of refractive index values in this range over this wavelength range provides that the improved SNR is available for a wide range of PPG measurements.

In accordance with one aspect of the present disclosure, a photoplethysmography device is provided. The photoplethysmography device includes: the adhesive patch, at least one optical source, at least one optical detector, and a mount. The mount is configured to support the at least one optical source and the at least one optical detector relative to the body tissue for providing optical coupling between the at least one optical source and the body tissue and/or between the at least one optical detector and the body tissue, via the transparent region of the adhesive patch, when the first surface of the adhesive patch is coupled to the body tissue and the second surface of the adhesive patch is coupled to the photoplethysmography device. Since the photoplethysmography device includes the adhesive patch, the photoplethysmography device benefits from the advantages of the adhesive patch described above.

In accordance with one aspect of the present disclosure, a patient monitoring system is provided. The patient monitoring system includes the photoplethysmography device, at least one processor, and a display device. The at least one processor is in communication with the at least one optical source and the at least one optical detector. The at least one processor is configured to:
control the at least one optical source to generate optical radiation;
receive electrical signals generated by the at least one optical detector in response to the generated optical radiation;
generate photoplethysmography measurement data based on the received electrical signals; and
output the photoplethysmography measurement data to the display device.

Since the patient monitoring system includes the photoplethysmography device that in-turn includes the adhesive patch, the photoplethysmography device benefits from the advantages of the adhesive patch described above.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating a first example of a photoplethysmography device 120 including an adhesive patch 100, in accordance with some aspects of the present disclosure.
Fig. 2 is a schematic diagram illustrating a second example of a photoplethysmography device 120 including an adhesive patch 100, in accordance with some aspects of the present disclosure.
Fig. 3 is a schematic diagram illustrating a third example of a photoplethysmography device 120 including an adhesive patch 100, in accordance with some aspects of the present disclosure.
Fig. 4 is a schematic diagram illustrating a fourth example of a photoplethysmography device 120 including an adhesive patch 100, in accordance with some aspects of the present disclosure.
Fig. 5 is a schematic diagram illustrating a first example of an adhesive patch 100, in accordance with some aspects of the present disclosure.
Fig. 6 is a schematic diagram illustrating a second example of an adhesive patch 200, in accordance with some aspects of the present disclosure.
Fig. 7 is a schematic diagram illustrating a third example of an adhesive patch 300, in accordance with some aspects of the present disclosure.
Fig. 8 is a schematic diagram illustrating a fourth example of an adhesive patch 400, in accordance with some aspects of the present disclosure.
Fig. 9 is a schematic diagram illustrating a fifth example of an adhesive patch 500, in accordance with some aspects of the present disclosure.
Fig. 10 is a schematic diagram illustrating a variant of a fifth example of an adhesive patch 500, in accordance with some aspects of the present disclosure.
Fig. 11 is a schematic diagram illustrating a sixth example of an adhesive patch 600, in accordance with some aspects of the present disclosure.
Fig. 12 is a schematic diagram illustrating a variant of a sixth example of an adhesive patch 600, in accordance with some aspects of the present disclosure.
Fig. 13 is a schematic diagram illustrating a seventh example of an adhesive patch 700, in accordance with some aspects of the present disclosure.
Fig. 14 is a schematic diagram illustrating a variant of a seventh example of an adhesive patch 700, in accordance with some aspects of the present disclosure.
Fig. 15 is a schematic diagram illustrating an example of a patient monitoring system 280, in accordance with some aspects of the present disclosure.

### DETAILED DESCRIPTION OF EMBIDIMENTS

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to an adhesive patch, or a photoplethysmography device, may be implemented in another adhesive patch, or a photoplethysmography device, respectively, in a corresponding manner.

In the following description, reference is made to adhesive patch for providing optical coupling between body tissue and a photoplethysmography device. In some examples, reference is made to the provision of optical coupling between a portion of a finger and a photoplethysmography device. It is however to be noted that, unless stated explicitly, the adhesive patch may be used to provide optical coupling between other types of body tissue and a photoplethysmography device. For instance, the adhesive patch may be used to provide optical coupling between a portion of a wrist, forehead, or a portion of an ear lobe, or a portion of a nostril, and a photoplethysmography device. In some examples reference is made to a photoplethysmography device that is suitable for coupling to a finger. It is however to be noted that, unless stated explicitly, the adhesive patch may be used to provide optical coupling between body tissue and photoplethysmography devices that are suitable for coupling to other regions of the body. For instance, the adhesive patch may be used to provide optical coupling between body tissue and photoplethysmography devices that are suitable for coupling to a portion of a wrist, forehead, or a portion of an ear lobe, or a portion of a nostril.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed by the one or more processors of the system disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations that are performed by the one or more processors.

As mentioned above, there remains a need to improve the optical coupling between body tissue and PPG devices in order to improve their performance.

Examples of the present disclosure relate to an adhesive patch 100, 200, 300, 400, 500, 600, 700 for providing optical coupling between body tissue 110 and a photoplethysmography device 120. The adhesive patch includes:
a first surface 130 for coupling to the body tissue 110, the first surface comprising an adhesive material for providing temporary coupling to the body tissue 110;
a second surface 140 for coupling to the photoplethysmography device 120, the second surface being arranged opposite the first surface; and
a transparent region 150;
wherein the transparent region 150 extends between the first surface 130 and the second surface 140 for providing the optical coupling between the body tissue 110 and the photoplethysmography device 120 when the first surface is coupled to the body tissue and the second surface is coupled to the photoplethysmography device.

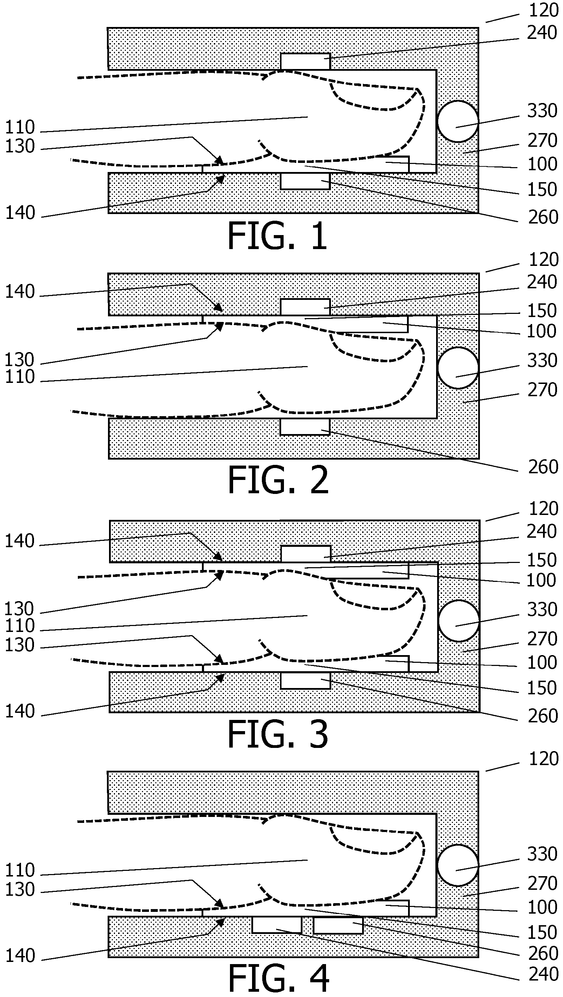

Fig. 1 is a schematic diagram illustrating a first example of a photoplethysmography device 120 including an adhesive patch 100, in accordance with some aspects of the present disclosure. The adhesive patch 100 provides optical coupling between body tissue 110, which in this example is a portion of a finger, and a photoplethysmography device 120, which in this example is a finger-mountable PPG device. The adhesive patch 100 includes a first surface 130 for coupling to the body tissue 110. The first surface 130 includes an adhesive material for providing temporary coupling to the body tissue 110.

The adhesive patch 100 illustrated in Fig. 1 also includes a second surface 140 for coupling to the photoplethysmography device 120. The second surface is arranged opposite the first surface. The adhesive patch 100 also includes a transparent region 150. The transparent region 150 extends between the first surface 130 and the second surface 140 for providing the optical coupling between the body tissue 110 and the photoplethysmography device 120 when the first surface is coupled to the body tissue and the second surface is coupled to the photoplethysmography device.

In the example illustrated in Fig. 1, the adhesive patch 100 provides optical coupling between body tissue 110 and a finger-mountable photoplethysmography device 120. In this example, the finger-mountable photoplethysmography device 120 includes a mount 270 and a hinge 330. The mount 330 is provided in two parts that are coupled via a hinge 330. The hinge 330 includes a spring that forces the two parts of the mount together such that when the finger is inserted between the two parts of the mount, the spring within the hinge applies pressure to the finger. The finger-mountable photoplethysmography device 120 also includes one or more optical sources 240 and one or more optical detectors 260. The one or more optical sources 240 and the one or more optical detectors 260 are arranged in an opposing configuration on the inner surfaces of the two parts of the mount in order to make optical transmission PPG measurements through a portion of the finger. When in-use, the transparent region 150 of the adhesive patch 100 is positioned over the at least one optical source 240.

As mentioned above, the adhesive patches disclosed herein may be used with various types of photoplethysmography devices. These include the finger-mountable photoplethysmography devices such as that illustrated in Fig. 1, other types of finger-mountable photoplethysmography devices, and also photoplethysmography devices that couple to other types of body tissue. In general, the adhesive patch may be used in photoplethysmography devices that perform PPG measurements in a transmission mode and/or in a reflectance mode. Fig. 2 - Fig. 4 illustrate further examples of finger-mountable photoplethysmography devices that include adhesive patches in accordance with some aspects of the present disclosure. It is noted that features that are illustrated in these examples that share the same label as Fig. 1 serve the same function as that described above and that a description of their function is omitted for brevity.

Fig. 2 is a schematic diagram illustrating a second example of a photoplethysmography device 120 including an adhesive patch 100, in accordance with some aspects of the present disclosure. The example illustrated in Fig. 2 differs from the example illustrated in Fig. 1 in that the adhesive patch 100 is arranged between the at least one optical source 240 of the photoplethysmography device 120 and the body tissue, rather than between the at least one optical detector 260 of the photoplethysmography device 120 and the body tissue. When in-use, the transparent region 150 of the adhesive patch 100 illustrated in Fig. 2 is positioned over the at least one optical source 240.

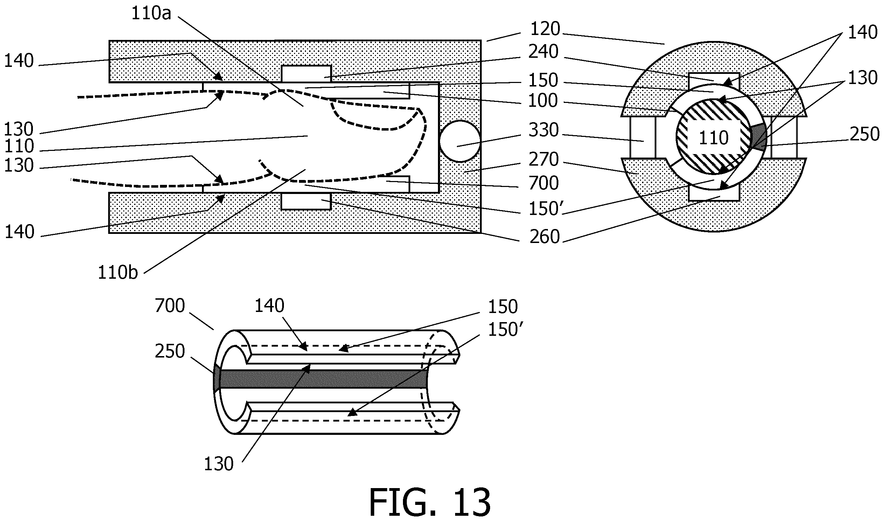

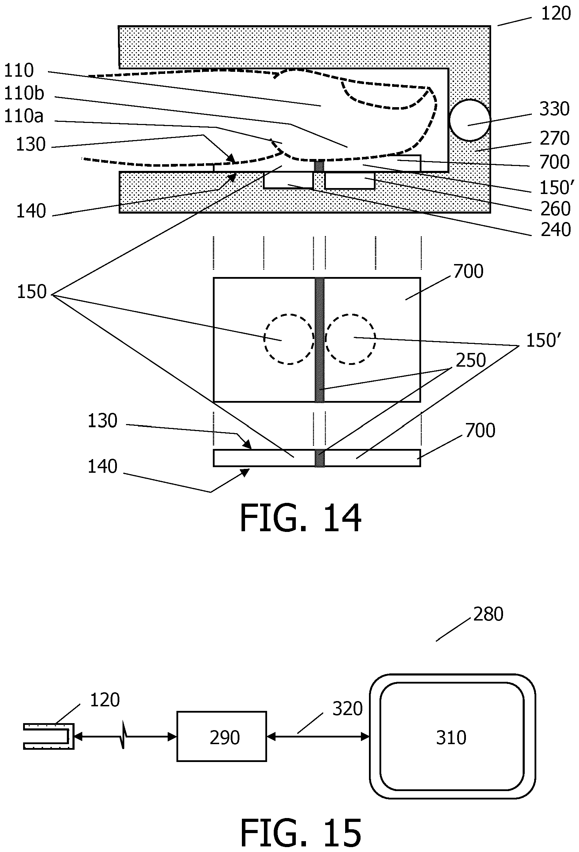

Fig. 3 is a schematic diagram illustrating a third example of a photoplethysmography device 120 including an adhesive patch 100, in accordance with some aspects of the present disclosure. The example illustrated in Fig. 2 differs from the example illustrated in Fig. 1 in that an additional adhesive patch 100 is arranged between the optical source 240 of the photoplethysmography device 120 and the body tissue. The example illustrated in Fig. 3 therefore represents the combination of the examples illustrated in Fig. 1 and Fig. 2. The adhesive patches 100 illustrated in Fig. 3 are separate items, although these may alternatively be combined into a single adhesive patch, as described later with reference to the examples illustrated in Fig. 13 and Fig. 14.

Fig. 4 is a schematic diagram illustrating a fourth example of a photoplethysmography device 120 including an adhesive patch 100, in accordance with some aspects of the present disclosure. The example illustrated in Fig. 4 differs from the example illustrated in Fig. 1 in that the at least one optical source 240 and the at least one optical detector 260 are arranged on the same side of the mount 270 for performing a reflectance PPG measurement on the body tissue 110. In the example illustrated in Fig. 4, when in-use, the transparent region 150 of the adhesive patch 100 is positioned over both the at least one optical source and over the at least one optical detector 260.

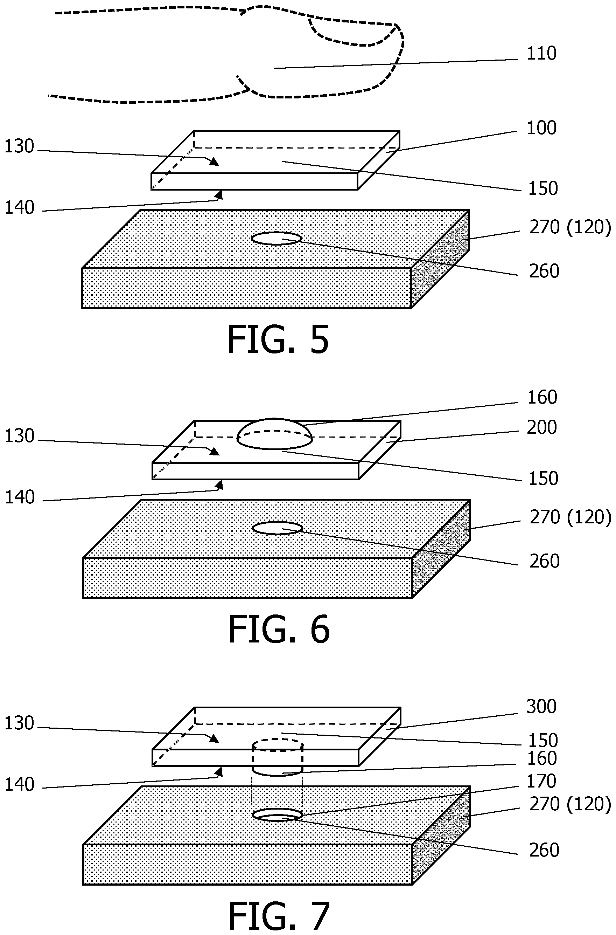

Various examples of adhesive patches are now described below with reference to Fig. 5 - Fig. 14.

Fig. 5 is a schematic diagram illustrating a first example of an adhesive patch 100, in accordance with some aspects of the present disclosure. As described above, the adhesive patch 100 may be used to provide optical coupling between body tissue 110, which in this example is a portion of a finger, and a photoplethysmography device 120. In the example illustrated in Fig. 5, only a part of the mount 270 of the photoplethysmography device 120 illustrated in Fig. 1, is shown, and this includes the at least one optical detector 260. The body tissue 110 and the part of the mount 270 are shown in Fig. 5 for illustration only, and it is to be appreciated that in an alternative example, the body tissue could be another type of body tissue, and that the part of the mount 270 could alternatively include the at least one optical source 240.

With reference to Fig. 5, the adhesive patch 100 includes a first surface 130 for coupling to the body tissue 110. The first surface 130 includes an adhesive material for providing temporary coupling to the body tissue 110. The coupling provided by the first surface 130 is temporary in the sense that when coupled to the surface of the skin, the adhesive patch 100 remains coupled to the skin for a period of some hours or more, and in that it may also be removed from the skin by force, e.g. by peeling the adhesive patch off the skin. After use, the adhesive patch may be disposed of. Thus, the adhesive patch may be referred-to as disposable. The adhesive material that provides the temporary coupling may be provided by various known materials. In general, the adhesive patch 100 may be formed from an adhesive material, and consequently the first surface 130 is inherently adhesive, or alternatively an adhesive material may be applied to a surface of an otherwise non-adhesive material in order to provide the adhesive patch 100. In the latter case, various adhesive materials may be used for this purpose, including for example Hydrogels, Silicone- or Acrylate-based hydrogels, and Silicone tape. In either case, it is noted that in general it is not required that the entirety of the first surface 130 comprises the adhesive material. For instance, the adhesive material may be arranged within zone, or in a pattern such as an array of spots, or a grid, over a portion of the first surface 130 such that one or more portions of the first surface 130 comprise the adhesive material.

With continued reference to Fig. 5, the adhesive patch 100 also includes a second surface 140 for coupling to the photoplethysmography device 120. The second surface may be configured for coupling to a portion of the photoplethysmography device 120 that includes one or more optical sources and/or one or more optical detectors. The second surface is arranged opposite the first surface. In the example illustrated in Fig. 5, the first and second surfaces are planar. In other examples at least one of the first and second surfaces are non-planar. For example, the first and/or second surface may be shaped in the form of a curve in order for the adhesive patch to conform to body tissue, such as a portion of a finger, for example. In some examples, the adhesive patch 100 has a flat shape in the resting state. In other examples, the adhesive patch 100 has a curved shape in the resting state. The latter may facilitate its attachment to a subject/s finger. In one example, the adhesive patch 100 is formed via 3D printing and is conformable such that it may conform to body tissue, such as a subject's finger.

In some examples, the second surface 140 comprises an adhesive material for providing temporary coupling to the photoplethysmography device 120. The adhesive material improves the stability of the optical coupling between the body tissue and the photoplethysmography device because it reduces relative movement between the body tissue and the photoplethysmography device when a subject moves. This reduces disruptions at this interface that might otherwise arise from a subject's movements and result in inaccurate PPG measurements or an absence of PPG measurements.

As also illustrated in Fig. 5, the adhesive patch 100 also includes a transparent region 150. The transparent region 150 extends between the first surface 130 and the second surface 140 for providing the optical coupling between the body tissue 110 and the photoplethysmography device 120 when the first surface is coupled to the body tissue and the second surface is coupled to the photoplethysmography device.

In the example illustrated in Fig. 5, the adhesive patch 100 is arranged between the at least one optical detector 260 of the photoplethysmography device 120 and the body tissue. When in-use, the transparent region 150 of the adhesive patch 100 is positioned over the at least one optical detector 260. The transparent region 150 of the adhesive patch 100 may alternatively or additionally be positioned over at least one optical source of the PPG device (not illustrated in Fig. 5). The transparent region 150 is transparent to a range of optical wavelengths that are used by the PPG device obtain PPG measurements. For example, the transparent region 150 may be transparent within the visible and/or infrared portions of the optical spectrum. In general, the transparent region may have a range of transmission values. For instance, in some examples, the transparent region has a negligible amount of absorption within the visible spectrum, and therefore a transmission value exceeding approximately 90% across the visible spectrum. In other examples, the transparent region may include a non-negligible amount of absorption. Thus, in these examples the transmission may have a value that is lower than 90%. In these examples, the transmission may also be higher than 10%. The transparent region 150 may also include an optical filter or alternatively be formed from a material that serves as an optical filter. The optical filter may attenuate optical radiation having optical wavelengths that are outside of a range of optical wavelengths emitted by an optical source of the PPG device, for example.

In some examples, the adhesive patch 100 is configured to be wrapped around at least a portion of a finger. The adhesive patch may have a size, a conformability, and a shape that facilitates its wrapping around a finger, for example. The adhesive patch may have a flat shape in the resting state, or a curved shape in the resting state, for example. The adhesive patch may therefore be used to obtain PPG measurements from a subject's finger.

In general, the adhesive patch may be formed from a variety of materials. In some examples, the adhesive patch is formed from a polymer material. For example, the adhesive patch may be formed from a polymer material such as polydimethylsiloxane "PDMS" or liquid silicone rubbers "LSR". Other examples of polymers that may be used include Hydrogels, Chitin, and Chitosan. In some examples, the adhesive patch is formed from a flexible material. Forming the adhesive patch from a flexible material allows it to conform to a surface of the body tissue. This improves the optical coupling between the body tissue and the photoplethysmography device because it limits the extent of an air gap that might otherwise form between the surface of the body tissue and the photoplethysmography device. Fresnel reflections may occur at this air gap and consequently reduce the amount of optical radiation emitted by an optical source of the photoplethysmography device that reaches an optical detector of the photoplethysmography device. Consequently, forming these elements from a flexible material serves to improve the SNR of PPG measurements that are acquired using the photoplethysmography device.

In some examples, the transparent region 150 of the adhesive patch comprises a refractive index value in the range from approximately 1.2 to 1.6. Body tissue typically has a refractive index of approximately 1.4. By providing the transparent region with a refractive index within this range, Fresnel reflection at the interface between the transparent region and body tissue is reduced. Fresnel reflection that might otherwise occur at this interface would reduce the amount of optical radiation emitted by an optical source of the photoplethysmography device that reaches an optical detector of the photoplethysmography device. Moreover, optical sources and optical detectors are typically formed from materials that have a refractive index of approximately 1.5, and so this range also serves to reduce Fresnel reflection at the interface between the transparent region and the optical source or detector. Consequently, providing the transparent region of the adhesive patch with a refractive index value in this range serves to improve the SNR of PPG measurements that are acquired using the photoplethysmography device.

In some examples, the adhesive patch comprises a refractive index value that is narrower than the range from 1.2 to 1.6. For instance, the refractive index value may be in a relatively narrower range of 1.4 to 1.5, or 1.5 to 1.6, or 1.2 to 1.6, or 1.2 to 1.5, or 1.3 to 1.6, or 1.3 to 1.5, or 1.3 to 1.4, or 1.4 to 1.5, or 1.35 to 1.45, or 1.3 to 1.6, or 1.4 to 1.6, or 1.5 to 1.6.

In some examples, the transparent region 150 comprises a refractive index in the range from 1.2 to 1.6 at one or more wavelengths in the range from approximately 520nm to 1000nm. As mentioned above, body tissue typically has a refractive index of approximately 1.4. By providing the transparent region with a refractive index within this range, Fresnel reflection at the interface between the transparent region and body tissue is reduced. This in-turn improves the SNR of PPG measurements that are acquired using the photoplethysmography device. Moreover, wavelengths in the range from 520nm to 1000nm are often used for PPG measurements, and so the use of refractive index values in this range over this wavelength range provides that the improved SNR is available for a wide range of PPG measurements.

In some examples, the adhesive material of the first surface 130 covers at least a portion of the transparent region 150. Alternatively or additionally, where present, the adhesive material of the second surface 140 may also cover at least a portion of the transparent region 150. Covering a portion these regions with adhesive material serves to limit the extent of an air gap that might otherwise form between the surface of the body tissue and these regions of the adhesive patch. Fresnel reflections that might otherwise occur at this air gap would reduce the amount of optical radiation emitted by an optical source of the photoplethysmography device that reaches an optical detector of the photoplethysmography device. Thus, covering a portion these regions with adhesive material serves to improve the SNR of PPG measurements that are acquired using the photoplethysmography device.

Various further examples of adhesive patches are now described below with reference to Fig. 6 - Fig. 14. It is noted that some of these patches share characteristics of the adhesive patches described above and that a further description of these features is omitted for brevity. For instance, features that are illustrated in these examples that share the same label as Fig. 5 serve the same function as that described above.

In one example, the first surface and/or the second surface of the adhesive patch 200 comprises a raised profile 160 within at least a portion of the transparent region 150. The raised profile 160 defines a lens for coupling optical radiation between the body tissue 110 and the photoplethysmography device 120, or vice versa, when the first surface 130 is coupled to the body tissue and the second surface 140 is coupled to the photoplethysmography device. In this example, the lens serves to improve the coupling of the optical radiation between the body tissue and the photoplethysmography device. The lens may concentrate optical radiation emitted by an optical source of the photoplethysmography device onto the body tissue and/or concentrate optical radiation emitted by the body tissue onto an optical detector of the photoplethysmography device, for example.

This example is described with reference to Fig. 6, which is a schematic diagram illustrating a second example of an adhesive patch 200, in accordance with some aspects of the present disclosure. In the example illustrated in Fig. 6, the first surface 130 comprises a raised profile 160 and the raised profile 160 defines a convex lens. Alternatively or additionally, the second surface 160 may define a (convex) lens. Thus, the transparent region 150 may include a plano-convex lens, or a biconvex lens. In other examples, the first and/or second surfaces may include a raised profile that defines another type of lens. For instance, a raised profile may alternatively be used to define a diffractive lens, or a Fresnel lens, or a Gradient-index "GRIN" lens, or a microlens.

In one example, the second surface 140 of the adhesive patch 300 comprises a raised profile 160 within the at least a portion of the transparent region. The raised profile is configured to mate with a corresponding recess 170 in a surface of the photoplethysmography device 120 for aligning the adhesive patch with the photoplethysmography device.

In this example, the mating that is provided by the raised profile and the corresponding recess serves to improve the stability of the optical coupling between the body tissue and the photoplethysmography device. Improving the stability of the optical coupling reduces disruptions at this interface that might otherwise arise from a subject's movements and result in inaccurate PPG measurements or an absence of PPG measurements.

This example is described with reference to Fig. 7, which is a schematic diagram illustrating a third example of an adhesive patch 300, in accordance with some aspects of the present disclosure. In the example illustrated in Fig. 7, the raised profile 160 is provided by a cylinder. The cylinder has a circular cross section that mates with a corresponding circular-shaped recess 170 in a surface of the photoplethysmography device 120. The cylinder also serves to guide optical radiation between the adhesive patch and the photoplethysmography device 120. For instance, in the example illustrated in Fig. 6 the cylinder guides optical radiation passing through the transparent region 150 onto the optical detector 260 of the photoplethysmography device 120. The use of raised profiles and corresponding recesses with other cross-sectional shapes is also contemplated. For instance, the raised profile may have a square shape, a rectangular shape, or indeed any other shape.

In a related example, the raised profile 160 of the adhesive patch 300 further defines a lens for coupling optical radiation between the body tissue 110 and the photoplethysmography device 120, or vice versa, when the first surface 130 is coupled to the body tissue and the second surface 140 is coupled to the photoplethysmography device.

In this example, the raised profile serves to improve the stability of the optical coupling, as well as to provide a lens that further improves the coupling of the optical radiation between the body tissue and the photoplethysmography device. With reference to Fig. 7, the lower surface of the cylindrical-shaped raised profile 160 of the second surface 140 may be provided with a profile similar to that of the raised profile 160 illustrated in Fig. 6 in order to likewise define a lens.

In one example, the second surface 140 of the adhesive patch 400 comprises one or more alignment features 180. The one or more alignment features are configured to mate with one or more corresponding alignment features 180' on the photoplethysmography device 120 for aligning the adhesive patch with the photoplethysmography device.

In this example, the mating that is provided by the alignment features and the alignment features serves to improve the stability of the optical coupling between the body tissue and the photoplethysmography device. Improving the stability of the optical coupling reduces disruptions at this interface that might otherwise arise from a subject's movements and result in inaccurate PPG measurements or an absence of PPG measurements.

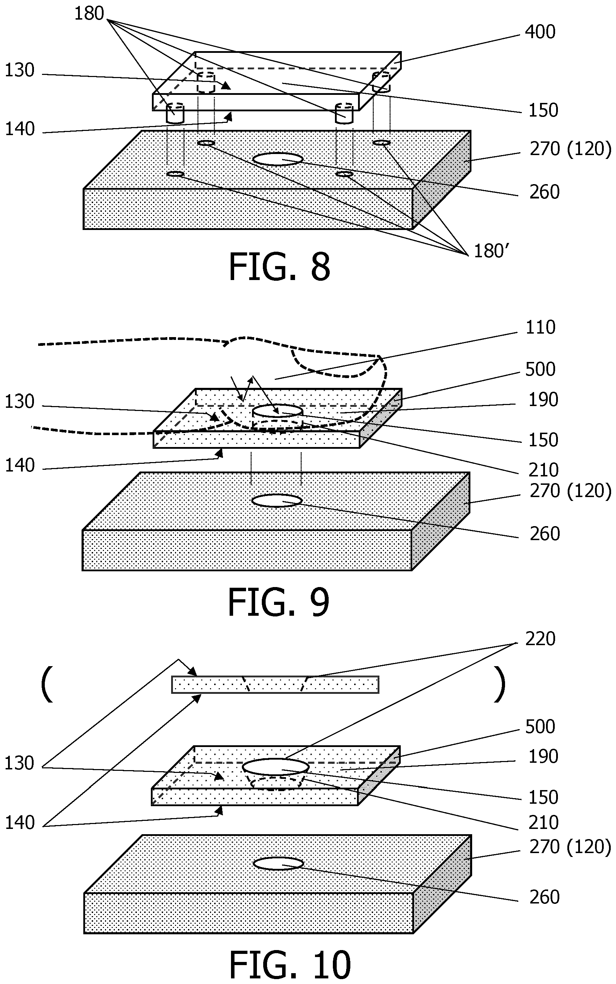

This example is described with reference to Fig. 8, which is a schematic diagram illustrating a fourth example of an adhesive patch 400, in accordance with some aspects of the present disclosure. In the example illustrated in Fig. 8, the alignment features 180 are protrusions in the second surface. The corresponding alignment features 180' on the photoplethysmography device 120 are recesses. In another configuration, recesses may serve as the alignment features 180, and protrusions may serve as the corresponding alignment features 180' on the photoplethysmography device 120. The alignment features 180 and the corresponding alignment features 180' illustrated in Fig. 8 have a circular cross-sectional shape. However it is to be appreciated that these may alternatively have other shapes. The alignment features 180 illustrated in Fig. 8 are formed from the same material as the adhesive patch 400. In alternative configurations, the alignment features 180 may be formed from a separate material.

In one example, the adhesive patch 500 includes a first border 190. The first border 190 at least partially surrounds the transparent region 150. The first border 190 comprises a reflective and/or scattering material for returning optical radiation incident on the first border to the body tissue when the first surface is coupled to the body tissue.

In this example, the first border recycles optical radiation that escapes from the skin by instead returning it to the skin and providing the optical radiation with another opportunity to reach an optical detector of the photoplethysmography device. The recycled optical radiation may therefore contribute to a PPG measurement, which improves the SNR of the PPG measurement.

This example is described with reference to Fig. 9, which is a schematic diagram illustrating a fifth example of an adhesive patch 500, in accordance with some aspects of the present disclosure. In general, the first border 190 may comprise a diffuse reflecting material or a specular reflecting material. For instance, the first border 190 may be formed from a white material, or by shiny material, respectively. In general, a white material provides diffuse reflectance, and a shiny material provides specular reflectance. Examples of diffuse scattering materials include white silicone material. Examples of specular reflecting materials include metals. If a scattering material is used, the scattering material may be a surface scattering material or a volume scattering material. A volume scattering material can be understood as a material with an ability to scatter optical radiation throughout its volume rather than only at its surface. It may comprise a medium with scattering media (e.g. particles or voids) embedded therein. The particles or voids disrupt the direct paths of the optical radiation, thereby scattering the optical radiation. Examples of volume scattering materials include a thermoplastic elastomer; a foam including scattering particles; and silicone including scattering particles. The scattering materials may be formed from a material such as titanium oxide.

In the example illustrated in Fig. 9, the first border 190 completely surrounds the transparent region 150 and forms the remainder of the adhesive patch 500 outside of the transparent region 150. In alternative configurations, the first border may have a more limited extent. For instance, the first border may be provided in the form of a strip that partially, or completely, surrounds the transparent region 130.

As mentioned above, the first border 190 recycles optical radiation that escapes from the skin by instead returning it to the skin and providing the optical radiation with another opportunity to reach an optical detector of the photoplethysmography device. This is illustrated in Fig. 9 via the ray paths of optical radiation travelling within the body tissue 110. Optical radiation that is generated by an optical source (not illustrated in Fig. 9) of the photoplethysmography device 120 is typically transmitted through the body tissue 110 via scattering. As indicated in Fig. 9, optical radiation travelling along the first ray path in the direction of the photoplethysmography device 120 is directed towards the mount 270 at an angle at which it would fall outside of the optical detector(s) 260. In the absence of the first border 190, this optical radiation would be incident on the mount 270 where it would be absorbed and consequently make no contribution to a PPG measurement. Instead, the first border 190 serves to reflect and/or scatter this optical radiation, returning it to the body tissue 110, as illustrated by the second ray path. The optical radiation is then scattered by the body tissue, and subsequently follows the third ray path illustrated in Fig. 9. This third ray path then continues through the optical window 150 to the optical detector 260 where the optical radiation is detected, and consequently it does contribute to a PPG measurement.

In a related example, the first border defines at least a portion of the first surface and/or the second surface. In the example illustrated in Fig. 9, the first border defines at least a portion of the first surface and the second surface. In other configurations the first border may be set back from the first and/or second surface. In the former case, the amount of recycled optical radiation may be improved. In the latter case, manufacturability of the adhesive patch may be improved.

In one example, the first border 190 comprises one or more inner sidewalls 210 contacting the transparent region 150, the one or more inner sidewalls defining an aperture for optical radiation passing through the transparent region. In this example, the aperture is a tapered aperture 220 having a relatively larger size at the first surface than at the second surface. Alternatively or additionally, the one or more inner sidewalls comprise a reflective material.

The tapered aperture, and likewise the reflective material serves to guide optical radiation between the body tissue and the photoplethysmography device. The tapered aperture may additionally serve to concentrate optical radiation onto an optical detector of the photoplethysmography device, or to provide a desired distribution in the body tissue of the optical radiation emitted by an optical source of the photoplethysmography device.

This example is described with reference to Fig. 9, and also with reference to Fig. 10, which is a schematic diagram illustrating a variant of a fifth example of an adhesive patch 500, in accordance with some aspects of the present disclosure. For instance, the inner sidewalls 210 of the adhesive patch 500 illustrated in Fig. 9 may include a reflective material. The reflective material may be provided by a layer comprising a metal or another type of material. It is noted that even in the absence of any reflective material, optical radiation may be guided by the one or more inner sidewalls 210 via total internal reflection. In the example illustrated in Fig. 10, a tapered aperture 220 is illustrated. The tapered aperture 220 is shown in a cross sectional view in the upper diagram in brackets in Fig. 10. The tapered aperture 220 may additionally include a reflective material to improve the guiding of optical radiation through the transparent region 150.

In one example, the adhesive patch 600 includes a second border 230. The second border 230 at least partially surrounds the transparent region 150 and/ or the first border 190. The second border 230 comprises an optically absorbing material.

The second border 230 serves to absorb optical radiation emitted by the optical source of the photoplethysmography device that either escapes from the body tissue, or does not pass through the body tissue at all. Some of the optical radiation that escapes from the body tissue includes pulsatile information relating to blood volume changes in the body tissue. This optical radiation has typically passed through body tissue in which there is a significant amount of blood flow. This may be described as functional optical radiation because it includes physiological information relating to the body. By contrast, some of the optical radiation that escapes from the body tissue does not include pulsatile information. This latter optical radiation has typically passed through portions of the body tissue such as bone, nail, and so forth, and in which there is an insignificant amount of blood flow. This may be described as non-functional optical radiation. Some optical radiation does not pass through the body tissue at all and is instead guided between the optical source and an optical detector of the photoplethysmography device, e.g. via reflection and/or scattering between a housing of the photoplethysmography device and a surface of the body tissue. This may be described as non-functional optical radiation because it does not include physiological information relating to the body. The detection of non-functional optical radiation is undesirable because it consumes the dynamic range of the optical detector, which in-turn degrades its sensitivity and/or places additional requirements on the processing of PPG measurements. The optically absorbing material in this aspect serves to absorb both the non-functional optical radiation that escapes from the body tissue, and the non-functional optical radiation that does not pass through the body tissue at all. This preserves the dynamic range of the optical detector, and in-turn avoids the degraded sensitivity and/or the additional processing requirements of the PPG measurements.

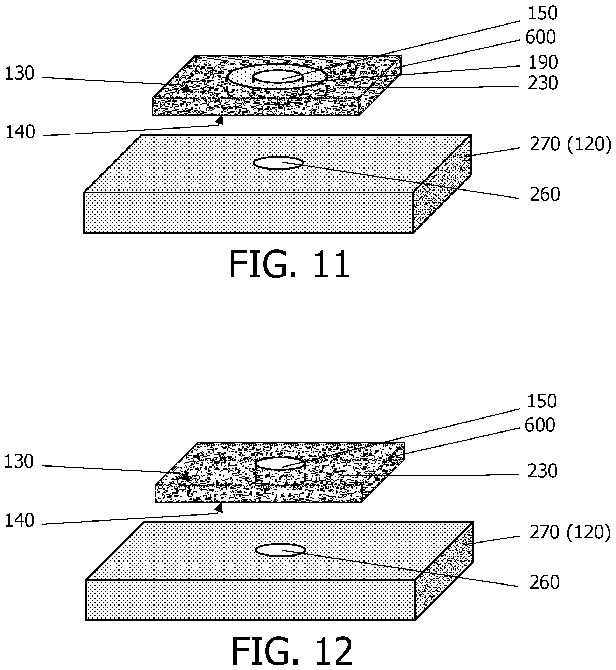

This example is described with reference to Fig. 11, and Fig. 12. Fig. 11 is a schematic diagram illustrating a sixth example of an adhesive patch 600, in accordance with some aspects of the present disclosure. In the example illustrated in Fig. 11, the second border 230 surrounds the first border 190. In this example, the second border 230 completely surrounds the first border 190 and forms the remainder of the adhesive patch 500 outside of the first border 190. In alternative configurations, the first border may have a more limited extent. For instance, the first border may be provided in the form of a strip that partially, or completely, surrounds the first border 190. In the example illustrated in Fig. 11, the second border 230 serves to absorb optical radiation that falls outside of the first border. Such optical radiation is likely to be non-functional optical radiation, or functional optical radiation that is sufficiently distant from the optical detector that it is unlikely to benefit from the recycling effect of the first border 190. Absorbing this optical radiation therefore reduces the impact of non-functional optical radiation described above.

Fig. 12 is a schematic diagram illustrating a variant of a sixth example of an adhesive patch 600, in accordance with some aspects of the present disclosure. The second border 230 in the example illustrated in Fig. 12 differs from the second border 230 illustrated in Fig. 11 in that in Fig. 12 the second border 230 is provided in the absence of the first border 190. In the example illustrated in Fig. 12, the second border 230 serves to absorb optical radiation that falls outside of the transparent region 190, and which might include non-functional optical radiation. Absorbing this optical radiation therefore reduces the impact of non-functional optical radiation described above.

In one example, the second border 230 defines at least a portion of the first surface and/or the second surface. In the example illustrated in Fig. 11 and Fig. 12, the second border 230 defines at least a portion of the first surface and the second surface. In other configurations the second border may be set back from the first and/or second surface. In the former case, the amount of recycled optical radiation may be improved. In the latter case, manufacturability of the adhesive patch may be improved.

In one example, the transparent region 150 of the adhesive patch 700 is configured to provide optical coupling between a first region 110a of the body tissue and an optical source 240 of the photoplethysmography device 120 when the first surface 130 is coupled to the body tissue and the second surface 140 is coupled to the photoplethysmography device. The adhesive patch further comprises:
a second transparent region 150'; and
an optically absorbing region 250;
wherein the second transparent region 150' extends between the first surface 130 and the second surface 140 for providing optical coupling between a second region 110b of the body tissue and an optical detector 260 of the photoplethysmography device when the first surface 130 is coupled to the body tissue and the second surface 140 is coupled to the photoplethysmography device; and
wherein the optically absorbing region 250 is arranged between the transparent region 150 and the second transparent region 150' for separating the optical coupling provided by the transparent region from the optical coupling provided by the second transparent region.

By separating the optical coupling that is provided by the transparent region from the optical coupling that is provided by the second transparent region, the optically absorbing region 250 reduces the amount of non-functional optical radiation that is detected by an optical detector of the photoplethysmography device.

This example is described with reference to Fig. 13, which is a schematic diagram illustrating a seventh example of an adhesive patch 700, in accordance with some aspects of the present disclosure. The example adhesive patch 700 is illustrated in isolation in the lower portion of Fig. 13. In the upper left and right portions of Fig. 13, the adhesive patch 700 is illustrated in two orthogonal cross-sectional views in combination with a finger-mountable photoplethysmography device 120 that is similar to that illustrated in Fig. 1. In the photoplethysmography device 120 illustrated in Fig. 13, the optical source(s) 240 and the optical detector(s) are arranged for making a transmission PPG measurement through the body tissue 110, which in this example is a portion of a finger.

In the example illustrated in Fig. 13, the adhesive patch 700 includes a transparent region 150 and also a second transparent region 150'. The optically absorbing region 250 is arranged between the transparent region 150 and the second transparent region 150' for separating the optical coupling provided by the transparent region from the optical coupling provided by the second transparent region. As may be appreciated, the optically absorbing region 250 absorbs optical radiation that is emitted by the optical source(s) 240 that might otherwise be guided to the optical detector(s) 260 via total internal reflection within the adhesive patch 700. Thus, the optically absorbing region 250 reduces the amount of non-functional optical radiation that is detected by the optical detector(s) 260.

In a related example, the second transparent region 150' comprises a refractive index value in the range from 1.2 to 1.6. As mentioned above, body tissue typically has a refractive index of approximately 1.4. Providing the transparent region with a refractive index within this range, similarly serves to reduce Fresnel reflection at the interface between the transparent region and tissue. This in-turn serves to improve the SNR of PPG measurements that are acquired using the photoplethysmography device.

With continued reference to the example illustrated in Fig. 13, the adhesive patch 700 is configured to be wrapped around at least a portion of a finger. The adhesive patch 700 is illustrated as having a curved shape in the resting state. This improves the ability of the adhesive patch 700 to conform to the shape of the finger. In alternative configurations, the adhesive patch 700 may have other shapes that facilitate its wrapping around at least a portion of a finger. For instance, the adhesive patch 700 may have a flat shape in the resting state. In one example, the adhesive patch 100 is formed via 3D printing and is conformable such that it may conform to body tissue, such as a portion of a subject's finger.

With continued reference to the example illustrated in Fig. 13, the adhesive patch 700 is configured to be wrapped around the at least a portion of a finger such that the transparent region 150 is arranged on one side of the finger and such that the second transparent region 150' is arranged on an opposing side of the finger for making an optical transmission measurement through the finger with the photoplethysmography device. The adhesive patch may therefore be used to obtain PPG measurements from a subject's finger in a transmissive mode. This may be arranged by providing a suitable distance between the transparent regions 150 and 150'.

Fig. 14 is a schematic diagram illustrating a variant of a seventh example of an adhesive patch 700, in accordance with some aspects of the present disclosure. The adhesive patch 700 illustrated in Fig. 14 differs from that illustrated in Fig. 13 in that the adhesive patch 700 illustrated in Fig. 14 is configured for use in acquiring reflectance PPG measurements. The adhesive patch 700 illustrated in Fig. 14 may be relatively smaller than that illustrated in Fig. 13 since a reflective PPG measurement does not require wrapping around a finger.

In some examples, at least one of the following is formed from a polymer material: the transparent region 150, the second transparent region 150', the first border 190, the second border 230. Polymer materials such as polydimethylsiloxane "PDMS" or liquid silicone rubbers "LSR" may be used, for example. Other examples of polymers that may be used include Hydrogels, Chitin, and Chitosan.

In some examples, at least one of the following is formed from a flexible material: the transparent region 150, second transparent region 150', the first border 190, the second border 230, absorbing region 250. Forming these elements from a flexible material allows them to conform to a surface of the body tissue. This improves the optical coupling between the body tissue and the photoplethysmography device because it limits the extent of an air gap that might otherwise form between the surface of the body tissue and the photoplethysmography device. Fresnel reflections may occur at this air gap and consequently reduce the amount of optical radiation emitted by an optical source of the photoplethysmography device that reaches an optical detector of the photoplethysmography device. Consequently, forming these elements from a flexible material serves to improve the SNR of PPG measurements that are acquired using the photoplethysmography device.

In one example, a photoplethysmography device 120, is provided. The photoplethysmography device 120 includes the adhesive patch described above, at least one optical source 240, at least one optical detector 260, and a mount 270. The mount 270 is configured to support the at least one optical source 240 and the at least one optical detector 260 relative to the body tissue 110 for providing optical coupling between the at least one optical source and the body tissue and/or between the at least one optical detector and the body tissue, via the transparent region 150 of the adhesive patch, when the first surface 130 of the adhesive patch is coupled to the body tissue and the second surface 140 of the adhesive patch is coupled to the photoplethysmography device.

Since the photoplethysmography device includes the adhesive patch, the photoplethysmography device benefits from the advantages of the adhesive patch described above.

Examples of the photoplethysmography device 120 are illustrated in the figures and described with reference to Fig. 1. As mentioned above, the photoplethysmography devices may be configured to acquire PPG measurements in a transmission mode and/or in a reflectance mode. The photoplethysmography device may be a finger-mountable photoplethysmography device, or it may be configured to mount to another type of body tissue, such as body tissue in a wrist, forehead, or an ear lobe, or a nostril, of a subject. Thus, the mount 270 may have a variety of configurations. The optical source(s) 240 may be provided by a variety of optical sources, including for example light emitting diodes "LEDs", lasers, and so forth. The optical source(s) may in general be configured to generate optical radiation within one or more wavelength intervals. For instance, the optical sources may be configured to generate optical radiation within a wavelength interval that corresponds to the red portion of the optical spectrum (e.g. approximately 660 nm/ 525 nm) and also within a wavelength interval that corresponds to the infrared portion of the optical spectrum (e.g. approximately 880 nm or 940 nm, respectively) in order to perform a blood oxygen saturation measurement. The optical detector(s) 260 may be provided by a variety of optical detectors, including for example photodiodes, photodetectors, and so forth.

In one example, a patient monitoring system 280 is provided. The patient monitoring system 280 includes the photoplethysmography device 120 described above, at least one processor 290, and a display device 310. The at least one processor 290 is in communication with the at least one optical source 240 and the at least one optical detector 260. The at least one processor 290 is configured to:
control the at least one optical source 240 to generate optical radiation;
receive electrical signals generated by the at least one optical detector 260 in response to the generated optical radiation;
generate photoplethysmography measurement data 320 based on the received electrical signals; and
output the photoplethysmography measurement data 320 to the display device 310.

Since the patient monitoring system includes the photoplethysmography device that in-turn includes the adhesive patch, the photoplethysmography device benefits from the advantages of the adhesive patch described above.

Fig. 15 is a schematic diagram illustrating an example of a patient monitoring system 280, in accordance with some aspects of the present disclosure. In the example illustrated in Fig. 15, the at least one processor 290 is illustrated as being remote with respect to the photoplethysmography device 120 and to the display device 310. However, it is to be appreciated that other combinations of these elements may be used. For instance, the at least one processor 290 may be included within the photoplethysmography device 120, or within the display device 310. The at least one processor 290 and the display device 310 may therefore be comprised in a single unit. As illustrated in Fig. 15, the processor(s) 290 is in communication with the photoplethysmography device 120 and the display device 310. This communication may be provided by any form of data communication, including via wired, or wireless, or optical fiber communication. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber.

The display device 310 illustrated in Fig. 15 may be any form of display device, such as a monitor, a virtual! augmented reality display device, a display of a mobile communication device such as a mobile telephone, a tablet, and so forth. Various known techniques may be used by the processor(s) 290 to generate photoplethysmography measurement data 320 based on the received electrical signals. For example, blood oxygen saturation measurement data may be derived from the ratio of pulse amplitudes of red and infrared PPG measurements (e.g. approximately 660 nm and approximately 880 nm or 940 nm, respectively) using the "ratio of ratios" technique. The photoplethysmography measurement data 320 may be outputted to the display device 310 in various ways, including in the form of a trace representing the measurement data over time.

The operations performed by the processor(s) may be stored on a computer-readable storage medium in the form of instructions which when executed by the one or more processors, cause the one or more processors to carry out the aforementioned operations.

The above examples are to be understood as illustrative of the present disclosure, and not restrictive. Further examples are also contemplated. For instance, the examples described in relation to an adhesive patch, may be included in a photoplethysmography device that includes the adhesive patch, or in a patient monitoring system that includes a photoplethysmography device that includes the adhesive patch, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. An adhesive patch (100, 200, 300, 400, 500, 600, 700) for providing optical coupling between body tissue (110) and a photoplethysmography device (120), the adhesive patch comprising:
a first surface (130) for coupling to the body tissue (110), the first surface comprising an adhesive material for providing temporary coupling to the body tissue (110);
a second surface (140) for coupling to the photoplethysmography device (120), the second surface being arranged opposite the first surface; and
a transparent region (150);
wherein the transparent region (150) extends between the first surface (130) and the second surface (140) for providing the optical coupling between the body tissue (110) and the photoplethysmography device (120) when the first surface is coupled to the body tissue and the second surface is coupled to the photoplethysmography device.

2. The adhesive patch according to claim 1, wherein the transparent region comprises a refractive index value in the range from approximately 1.2 to 1.6.

3. The adhesive patch (200) according to claim 1 or claim 2, wherein the first surface and/or the second surface comprises a raised profile (160) within at least a portion of the transparent region (150); and
wherein the raised profile (160) defines a lens for coupling optical radiation between the body tissue (110) and the photoplethysmography device (120), or vice versa, when the first surface (130) is coupled to the body tissue and the second surface (140) is coupled to the photoplethysmography device.

4. The adhesive patch (300) according to any previous claim, wherein the second surface comprises a raised profile (160) within the at least a portion of the transparent region; and
wherein the raised profile is configured to mate with a corresponding recess (170) in a surface of the photoplethysmography device (120) for aligning the adhesive patch with the photoplethysmography device.

5. The adhesive patch (400) according to any previous claim, wherein the second surface (140) comprises one or more alignment features (180); and
wherein the one or more alignment features are configured to mate with one or more corresponding alignment features (180') on the photoplethysmography device (120) for aligning the adhesive patch with the photoplethysmography device.

6. The adhesive patch (500) according to any previous claim, further comprising a first border (190);
wherein the first border (190) at least partially surrounds the transparent region (150);
wherein the first border (190) comprises a reflective and/or scattering material for returning optical radiation incident on the first border to the body tissue when the first surface is coupled to the body tissue.

7. The adhesive patch according to claim 6, wherein the first border comprises one or more inner sidewalls (210) contacting the transparent region (150), the one or more inner sidewalls defining an aperture for optical radiation passing through the transparent region; and
wherein the aperture is a tapered aperture (220) having a relatively larger size at the first surface than at the second surface;
and/or
wherein the one or more inner sidewalls comprise a reflective material.

8. The adhesive patch (600) according to any previous claim, further comprising a second border (230);
wherein the second border (230) at least partially surrounds the transparent region (150) and/ or the first border (190); and
wherein the second border (230) comprises an optically absorbing material.

9. The adhesive patch (700) according to any previous claim, wherein the transparent region (150) is configured to provide optical coupling between a first region (110a) of the body tissue and an optical source (240) of the photoplethysmography device (120) when the first surface (130) is coupled to the body tissue and the second surface (140) is coupled to the photoplethysmography device; and wherein the adhesive patch further comprises:
a second transparent region (150'); and
an optically absorbing region (250);
wherein the second transparent region (150') extends between the first surface (130) and the second surface (140) for providing optical coupling between a second region (110b) of the body tissue and an optical detector (260) of the photoplethysmography device when the first surface (130) is coupled to the body tissue and the second surface (140) is coupled to the photoplethysmography device; and
wherein the optically absorbing region (250) is arranged between the transparent region (150) and the second transparent region (150') for separating the optical coupling provided by the transparent region from the optical coupling provided by the second transparent region.

10. The adhesive patch according to any previous claim, wherein the adhesive patch is configured to be wrapped around at least a portion of a finger.

11. The adhesive patch according to claim 10, when dependent on claim 9, wherein the adhesive patch is configured to be wrapped around the at least a portion of a finger such that the transparent region (150) is arranged on one side of the finger and such that the second transparent region (150') is arranged on an opposing side of the finger for making an optical transmission measurement through the finger with the photoplethysmography device.

12. The adhesive patch according to any previous claim, wherein the second surface (140) comprises an adhesive material for providing temporary coupling to the photoplethysmography device (120).

13. The adhesive patch according to claim 1, wherein the transparent region (150) comprises a refractive index in the range from 1.2 to 1.6 at one or more wavelengths in the range from approximately 520nm to 1000nm.

14. A photoplethysmography device (120), comprising:
the adhesive patch according to any one of claims 1 - 13;
at least one optical source (240);
at least one optical detector (260); and
a mount (270);
wherein the mount (270) is configured to support the at least one optical source (240) and the at least one optical detector (260) relative to the body tissue (110)for providing optical coupling between the at least one optical source and the body tissue and/or between the at least one optical detector and the body tissue, via the transparent region (150) of the adhesive patch, when the first surface (130) of the adhesive patch is coupled to the body tissue and the second surface (140) of the adhesive patch is coupled to the photoplethysmography device.

15. A patient monitoring system (280) comprising:
the photoplethysmography device (120) according to claim 14;
at least one processor (290); and
a display device (310);
wherein the at least one processor (290) is in communication with the at least one optical source (240) and the at least one optical detector (260); and
wherein the at least one processor (290) is configured to:
control the at least one optical source (240) to generate optical radiation;
receive electrical signals generated by the at least one optical detector (260) in response to the generated optical radiation;
generate photoplethysmography measurement data (320) based on the received electrical signals; and
output the photoplethysmography measurement data (320) to the display device (310).
